(19) Europäisches Patentamt
European Patent Office
Office européen des brevets

(11) Publication number: **0 545 892 A1**

(12) **EUROPEAN PATENT APPLICATION**

(21) Application number: **93100641.5**

(22) Date of filing: **21.07.88**

(51) Int. Cl.[5]: **C07D 493/08**, C07D 497/08, C07D 495/08, A01N 43/90, A61K 31/335, A61K 31/385, A61K 31/39, //(C07D493/08, 319:00,319:00),(C07D497/08, 327:00,327:00),(C07D497/08, 339:00,327:00),(C07D495/08, 339:00,339:00),(C07D493/08, 319:00,311:00),(C07D497/08, 335:00,327:00)

This application was filed on 18 - 01 - 1993 as a divisional application to the application mentioned under INID code 60.

(30) Priority: **22.07.87 GB 8717274**
**07.10.87 GB 8723488**
**26.04.88 GB 8809851**

(43) Date of publication of application: **09.06.93 Bulletin 93/23**

(60) Publication number of the earlier application in accordance with Art.76 EPC: **0 300 797**

(84) Designated Contracting States: **AT BE CH DE ES FR GB GR IT LI LU NL SE**

(71) Applicant: **ROUSSEL-UCLAF**
**35, Boulevard des Invalides**
**F-75007 Paris(FR)**

(72) Inventor: **Weston, John Bernard, Roussel-Uclaf**
**102,111, route de Noisy-B.P.9**
**F-93230 Romainville(FR)**
Inventor: **Larkin, John Patrick, Roussel-Uclaf**
**102,111, route de Noisy-B.P.9**
**F-93230 Romainville(FR)**
Inventor: **Smith, Ian Harold**
**The Wellcome Research Laboratories, Langley Court**
**Beckenham, Kent BR3 3BS(GB)**

(74) Representative: **Vieillefosse, Jean-Claude et al**
**Roussel-Uclaf 111, route de Noisy B.P. 9**
**F-93230 Romainville (FR)**

(54) **Pesticidal compounds.**

(57) Compounds of the formula

EP 0 545 892 A1

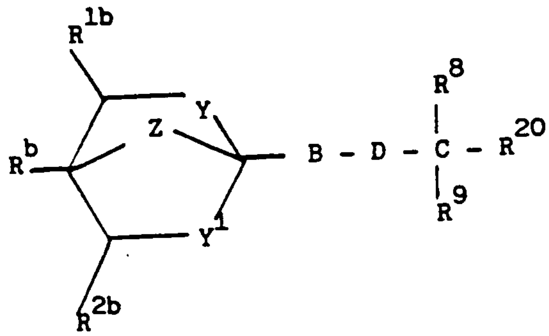

(IC)

wherein $R^b$ is a $C_{2-10}$ non aromatic hydrocarbyl group optionally substituted by cyano, halo, alkoxy, or a group $S(O)_mbR^{3b}$ or $R^b$ is phenyl optionally substituted by alkoxy, alkyl, alkynyl, halo, haloalkyl, cyano or a group $S(O)$-$_mbR^{3b}$ as defined hereinbefore;

$R^{1b}$ and $R^{2b}$ may be the same or different, and each is hydrogen, halo, or a aliphatic group optionally substituted by halo, cyano, carbalkoxy, or alkoxy; a group $S(O)_{m'}R^4$ and $R^4$ is alkyl; alkynyl, cyano, gem dimethyl, or carbalkoxy, or $R^{1b}$ and $R^b$ and the carbon atoms to which they are attached form a carbocyclic ring optionally substituted,

$R^8$, $R^9$ and $R^{20}$ may be the same or different and are selected from chloro, bromo, methoxy or methyl optionally substituted by methoxy or fluoro;

B is a single bond, methylene or a $C_{2-6}$ aliphatic chain which may contain one or two heteroatoms and/or double bonds but not triple bonds interspersed in the chain and which may be substituted by one to four substituents which may be the same or different and are each independently selected from hydroxy, oxo, halo, alkyl, alkoxy, acyloxy, epoxy, a alkylidene group, a carbalkoxy group, haloalkyl or cyano;

D is a single bond or a group $CH_2O$, $CH_2S(O)_n$ wherein n is 0, 1 or 2, or D is a 1,2 cyclopropyl group;

Y and $Y^1$ are the same or different and are each selected from oxygen and $S(O)_{n'}$ wherein n' is 0, 1 or 2; and Z is $CH_2CH_2$, $CH_2O$ or $CH_2S(O)_{n''}$ wherein n'' is 0, 1 or 2 provided that B cannot be a single bond or methylene group when D is a single bond, which have pesticidal activity, particularly against anthropod pests.

The present invention relates to novel chemical compounds having pesticidal activity, to methods for their preparation, to compositions containing them and to their use in the control of pests. More particularly the invention relates to a class of heterobicycloalkanes.

The use of certain pesticidally active 2,6,7-trioxabicyclo[2.2.2]octanes is disclosed in European Patent Applications Nos. 152229, 211598, 216625, 216624, US Patent 3686224 and J.Agric.Food Chem. 33, 976 (1985). It has now been discovered that derivatives of these compounds have interesting pesticidal activity.

Accordingly, the present invention provides a compound of the formula (IC)

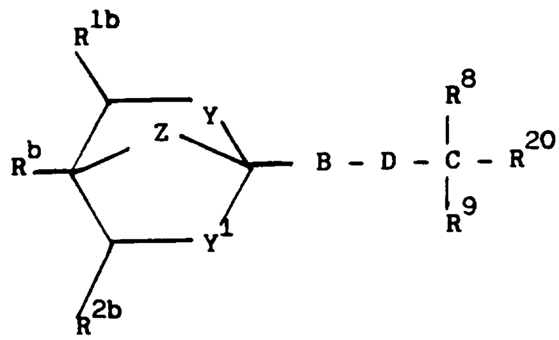

(IC)

wherein $R^b$ is a $C_{2-10}$ non-aromatic hydrocarbyl group optionally substituted by cyano, halo, $C_{1-4}$ alkoxy, or a group $S(O)_mbR^{3b}$ where $R^{3b}$ is $C_{1-4}$ alkyl and $m^b$ is 0, 1 or 2, or $R^b$ is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mbR^{3b}$ as defined hereinbefore;

$R^{1b}$ and $R^{2b}$ may be the same or different, and each is hydrogen, halo, or a $C_{1-3}$ aliphatic group optionally substituted by halo, cyano, $C_{1-5}$ carbalkoxy, or $C_{1-4}$ alkoxy; a group $S(O)_{m'}R^4$ wherein m' is 0, 1 or 2 and $R^4$ is $C_{1-4}$ alkyl; $C_{2-3}$ alkynyl, cyano, gem dimethyl, or $C_{1-5}$ carbalkoxy, or $R^{1b}$ and $R^b$ and the carbon atoms to which they are attached form a $C_{5-7}$ carbocyclic ring optionally substituted by halo, $C_{1-3}$ alkyl or alkoxy or $C_{2-3}$ alkenyl;

$R^8$, $R^9$ and $R^{20}$ may be the same or different and are selected from chloro, bromo, methoxy or methyl optionally substituted by methoxy or fluoro;

B is a single bond, methylene or a $C_{2-6}$ aliphatic chain which may contain one or two heteroatoms and/or double bonds but not triple bonds interspersed in the chain and which may be substituted by one to four substituents which may be the same or different and are each independently selected from hydroxy, oxo, halo, $C_{1-4}$ alkyl, $C_{1-4}$ alkoxy, $C_{1-4}$ acyloxy, epoxy, a $C_{1-4}$ alkylidene group, a $C_{1-6}$ carbalkoxy group, $C_{1-4}$ haloalkyl or cyano;

D is a single bond or a group $CH_2O$, $CH_2S(O)_n$ wherein n is 0,1 or 2,or D is a 1,2 cyclopropyl group;

Y and $Y^1$ are the same or different and are each selected from oxygen and $S(O)_{n'}$ wherein n' is 0, 1 or 2; and Z is $CH_2CH_2$, $CH_2O$ or $CH_2S(O)_{n''}$ wherein n'' is 0, 1 or 2 provided that B cannot be a single bond or methylene group when D is a single bond.

Suitably $R^b$ is propyl, butyl, pentyl, $C_{2-5}$ alkenyl or alkynyl, $C_{5-7}$ cycloalkyl or phenyl each optionally substituted by fluoro, chloro or bromo. Most suitably $R^b$ is n-propyl,n-butyl, i-butyl, sec-butyl, t-butyl, phenyl, cyclopentyl or cyclohexyl and preferably $R^b$ is n-propyl, n-butyl, i-butyl, or t-butyl.

Suitably $R^{1b}$ is hydrogen, cyano, methyl or ethyl each optionally substituted by cyano, methoxy, methylthio, chloro, bromo or fluoro. Most suitably $R^{1b}$ is hydrogen, methyl, cyano, trifluoromethyl or ethyl. Preferably $R^{1b}$ is hydrogen, methyl, cyano or trifluoromethyl.

Suitably $R^{2b}$ is hydrogen, cyano, methyl or trifluoromethyl. Most suitably $R^{2b}$ is hydrogen or methyl. Preferably $R^{2b}$ is hydrogen.

Suitable heteroatoms for inclusion in B include oxygen, nitrogen and sulphur, the sulphur being optionally oxidised as the sulphoxide or sulphone.

Suitably B is a group $(CH_2)_2$ or -CH=CH-.

Suitably D is a single bond.

Suitably Z is $CH_2S$ or $CH_2O$.

Suitably Y and $Y^1$ are both selected from oxygen or sulphur.

The compounds of the formula (IC) may exist in a number of isomeric forms. The present invention provides individual isomers of compounds of the formula (IC) and mixtures thereof. The present invention also encompasses compounds of the formula (IC) containing radioisotopes, particularly those in which one

carbon atom is $C^{14}$ or one to three hydrogen atoms are replaced by tritium.

Preferred compounds of the present invention include:

4-t-Butyl-1-(3,3-dimethylbutyl)-2,6,7-trioxabicyclo[2.2.2]octane

1-(3,3-Dimethylbutyl)-4-propyl-2,6,7-trioxabicyclo[2.2.2]octane-3-carbonitrile

1-[(E)-3,3-Dimethylbut-1-enyl]-4-propyl-2,6,7-trioxabicyclo[2.2.2]octane-3- carbonitrile

1-(t-Butylthiomethyl)-4-propyl-2,6,7-trioxabicyclo[2.2.2]octane-3-carbonitrile

1-[z)-1-Fluoro-3,3-dimethylbut-1-enyl]-4-propyl-2,6,7-trioxabicyclo[2.2.2]octane-3-carbonitrile

By the term "hydrocarbyl" group is meant alkyl, alkenyl (including cyclic alkyl and alkenyl, and alkyl and alkenyl substituted by cyclic alkyl and alkenyl), alkynyl, aryl and aralkyl groups. "Hydrocarbyloxy" means a hydrocarbyl group as defined where linked to oxygen.

By the term "aliphatic" group is meant an alkyl, alkenyl or alkynyl group.

By the term "halo" is meant fluoro, chloro, bromo or iodo.

In a further aspect, the present invention provides a process for the preparation of a compound of the formula (IC). The process for the preparation of a compound of the formula (IC) may be any method known in the art for preparing analogous compounds, for example :

(i) when Y and $Y^1$ are oxygen and Z is $CH_2O$:

a) by the cyclisation of a compound of the formula (II):

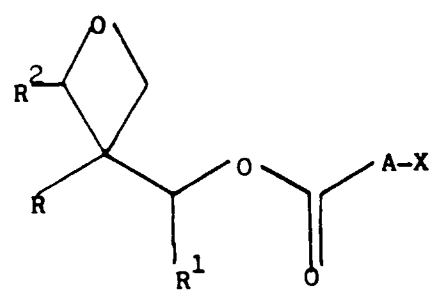

(II)

wherein $R^b$ to $R^{2b}$, $R^8$, $R^9$, $R^{20}$, B and D are as hereinbefore defined, in the presence of an acid catalyst. Boron trifluoride etherate is a particularly preferred acid catalyst for this cyclisation which will normally be carried out in an inert solvent, such as a halogenated hydrocarbon, conveniently dichloromethane, at or below ambient temperature, for example between -100 and 50°C and conveniently between -70 and -25°C.

The compounds of the formula (II) may be prepared by the reaction of compounds of the formulae (III) and (IV):

(III)

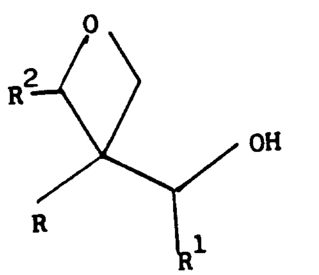

(IV)

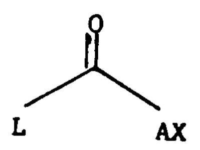

where $R^b$ to $R^{2b}$, $R^8$, $R^9$, $R^{20}$, B and D are as hereinbefore defined and L is a leaving group such as halo or hydroxy. This reaction conveniently takes place under conditions well known to those skilled in the art, for example when L is halo in an inert solvent in the presence of base at a non-extreme temperature and when L is hydroxy in an inert solvent in the presence of a condensing agent at a non extreme temperature. When L is halo, halogenated hydrocarbons, such as dichloromethane, are particularly suitable inert solvents and pyridine is a preferred base; when L is hydroxy, dimethylformamide is a suitable solvent, dicyclohexylcarbodiimide is a preferred condensing agent; and the reaction will conveniently be carried out at between -50 and 100°C, preferably between 0 and 25°C.

The compounds of the formula III may be prepared as described in copending European Patent Applications Nos. 211598 and 216624. The compounds of the formula (IV) may be prepared by methods well known to those skilled in the art.

(ii) when n is O

$Y^1$ = O or S

Y = O or S

Z = $CH_2S$ or $CH_2O$

by the reaction of a compound of the formula (XIV) with a compound of the formula

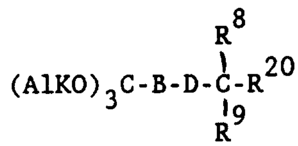

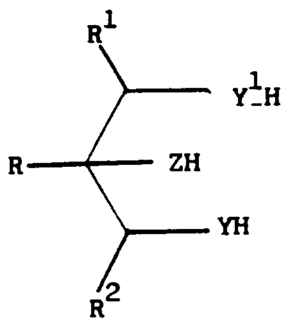

(XIV)

wherein $R^b$ $R^{1b}$, $R^{2b}$, $R^8$, $R^9$, $R^{20}$, B, D, Y, $Y^1$ and Z are as hereinbefore defined and Alk is a $C_{1-4}$ alkyl group. The condensation takes place in the presence of an acid catalyst for example a mineral acid such as concentrated hydrochloric acid or boron trifluoride etherate and/or p-toluenesulphonic. The reaction is conveniently carried out without a solvent, but an inert solvent, conveniently a chlorinated hydrocarbon such as dichloromethane may be added. The reaction can also be carried out in methanol containing hydrogen chloride. The reaction is conveniently carried out at a non-extreme temperature, for example between -70°C and 150°C and normally between -10°C and 150°C. The compounds of the formula (XIV) may be prepared as described in European Patent Application No 216624, or as described in appendix 1. Compounds of the formula (XIV) wherein $Y=Y^1=S$, $Z=CH_2S$ and $R^1$ = $R^2$ = H may also be prepared by the method described by G. R. Franzen and G. Binsch, J.Amer.Chem.Soc., 1973, 95, 175 and D.J.martin and C.R.Creco, J.Org.Chem., 1968, 33, 1275. The compounds of the formula $(AlkO)_3CAX$ may be prepared by a general procedure for the synthesis of orthoesters and is described by S.M.McElvain and R.E.Stam, J.Amer.Chem.Soc., 1955, 77, 4571:

(iii)when Z = $CH_2S$ or $CH_2O$ and Y and $Y^1$ are sulphur by reaction of a compound of the formula (XV)

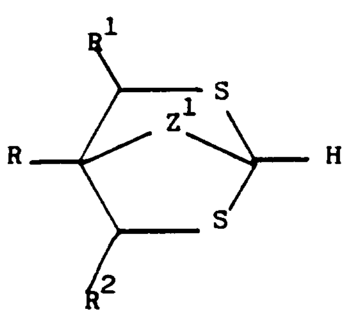

(XV)

with a compound

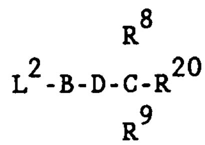

wherein Rb to $R^{2b}$, $R^8$, $R^9$, $R^{20}$, B and D are as hereinbefore defined, $Z^1$ is $CH_2S$ or $CH_2O$ and $L^2$ is a leaving group eg.halo. The reaction is suitably carried out in the presence of a strong base, such as butyllithium, in an inert solvent, such as an ether and conveniently tetrahydrofuran at a non-extreme temperature, such as between 70° and 30°C. The compound of the formula (XV) can be prepared by the reaction of an analogous compound of the formula (XIV) with $HC(OAlk)_3$ under the conditions described for reaction (ii) above.

(iv) when Y is 0 and $Y^1$ is O and Z is $CH_2CH_2$ by the reaction of a compound of the formula (XVI) with acid

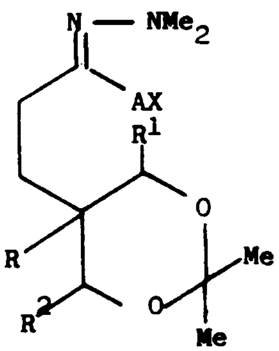

(XVI)

wherein $R^b$, $R^{1b}$, $R^{2b}$, $R^8$, $R^9$, $R^{20}$, B and D are as hereinbefore defined.

The reaction is carried out in acidic conditions, conveniently silica gel, followed by dilute hydrochloric acid at a non-extreme temperature, i.e. between 0° and 100° and conveniently ambient temperature i.e. between 20° and 30°. The compounds of the formula (XVI) may be prepared as illustrated in Appendix 2 herein.

(v) By the interconversion of compounds of the formula (IC), for example

(a) when it is required to prepare a compound of the formula (IC) wherein B is saturated or contains a double bond by the reduction of the corresponding compound containing a double or triple bond respectively. This reaction conveniently takes place by hydrogenation in the presence of a catalyst, for example palladium on charcoal, or, when a triple bond is being reduced and it is required to stop the reduction once a double bond is formed and not proceed to the fully saturated compound, in the presence of such a catalyst poisoned by, for example, barium sulphate. The reaction is conveniently carried out in a suitable solvent for hydrogenation experiments such as methanol or ethyl acetate.

The reaction is usually carried out at a non-extreme temperature, for example between 5° and 50°C and normally at 25°C.

Novel chemical intermediates also form an important aspect of the present invention. Preferred intermediates include those of the formula (II), (XIV) and (XV).

The compounds of formula (IC) may be used to control pests such as arthropods e.g. insect and acarine pests, and helminths, i.e. nematodes. Thus, the present invention provides a method for the control of arthropods and/or helminths which comprises administering to the arthropod and/or helminth or to their environment an arthropodically effective amount of a compound of the formula (IC). The present invention also provides a method for the control and/or eradication of arthropod and/or helminth infestations of animals (including humans) and/or of plants, (including trees) and/or stored products which comprises administering to the animal or locus an effective amount of a compound of the formula (IC). The present invention further provides for the compounds of the formula (IC) for use in human and veterinary medicine, in public health control and in agriculture for the control of arthropod and/or helminth pests.

The compounds of formula (IC) are of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole

crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids).

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compounds of general formula (IC) are of particular value in the control of arthropods or helminths which are injurious to, or spread or act as vectors of diseases in man and domestic animals, for example those hereinbefore mentioned, and more especially in the control of ticks, mites, lice, fleas, midges and biting, nuisance and myiasis flies.

The compounds of Formula (IC) may be used for such purposes by application of the compounds themselves or in diluted form in known fashion as a dip, spray, fog, lacquer, foam, dust, powder, aqueous suspension, paste, gel, cream, shampoo, grease, combustible solid, vapourising mat, combustible coil, bait, dietary supplement, wettable powder, granule, aerosol, emulsifiable concentrate, oil suspensions, oil solutions, pressure-pack, impregnated article, pour on formulation or other standard formulations well known to those skilled in the art. Dip concentrates are not applied per se, but diluted with water and the animals immersed in a dipping bath containing the dip wash. Sprays may be applied by hand or by means of a spray race or arch. The animal, soil, plant or surface being treated may be saturated with the spray by means of high volume application or superficially coated with the spray by means of light or ultra low volume application. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are dispensed as a unit of liquid of small volume on to the backs of animals such that all or most of the liquid is retained on the animals.

The compounds of Formula (IC) may be prepared either as formulations ready for use on the animals, plants or surface or as formulations requiring dilution prior to application, but both types of formulation comprise a compound of Formula (IC) in intimate admixture with one or more carriers or diluents. The carriers may be liquid, solid or gaseous or comprise mixtures of such substances, and the compound of Formula (IC) may be present in a concentration of from 0.025 to 99% w/v depending upon whether the formulation requires further dilution.

Dusts, powders and granules and other solid formulations comprise the compound of Formula (IC) in intimate admixture with a powdered solid inert carrier for example suitable clays, kaolin, bentonite, attapulgite, adsorbent carbon black, talc, mica, chalk, gypsum, tricalcium phosphate, powdered cork, magnesium siliate, vegetable carriers, starch and diatomaceous earths. Such solid formulations are generally prepared by impregnating the solid diluents with solutions of the compound of formula (IC) in volatile solvents, evaporating the solvents and, if desired grinding the products so as to obtain powders and, if desired, granulating, compacting or encapsulating the products.

Sprays of a compound of Formula (IC) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates. Cationic emulsifiers include benzalkonium chloride and quaternary ammonium ethosuphates.

Amphoteric emulsifiers include carboxymethylated oleic imidazoline and alkyl dimethyl betain.

Vaporising mats normally comprise cotton and cellulose mix compressed into a board of approximately 35 x 22 x 3mm dimensions, treated with up to 0.3ml of concentrate comprising the active ingredient in an organic solvent and optionally an antioxidant, dye and perfume. The insecticide is vaporised using a heat source such as an electrically operated mat heater.

Combustible solids normally comprise of wood powder and binder mixed with the active ingredient and formed into shaped (usually coiled) strips. Dye and fungicide may also be added.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 5 to 95% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of Formula (IC) in intimate admixture with a dispersing agent and one or more surface active agents.

Aqueous suspensions of a compound of Formula (IC) may comprise a suspension in water together with suspending, stabilizing or other agents. The suspensions or solutions may be applied per se or in a diluted form in known fashion.

Greases (or ointments) may be prepared from vegetable oils, synthetic esters of fatty acids or wool fat together with an inert base such as soft paraffin. A compound of Formula (IC) is preferably distributed uniformly through the mixture in solution or suspension. Greases may also be made from emulsifiable concentrates by diluting them with an ointment base.

Pastes and shampoos are also semi-solid preparations in which a compound of Formula (IC) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of Formula (IC) required for treatment.

Aerosol sprays may be prepared as a simple solution of the active ingredient in the aerosol propellant and co-solvent such as halogenated alkanes and the solvents referred to above, respectively. Pour-on formulations may be made as a solution or suspension of a compound of Formula (IC) in a liquid medium. An avian or mammal host may also be protected against infestation of acarine ectoparasites by means of carrying a suitably-moulded, shaped plastics article impregnated with a compound of Formula (IC). Such articles include impregnated collars, tags, bands, sheets and strips suitably attached to appropriate parts of the body. Suitably the plastics material is a polyvinyl chloride (PVC).

The concentration of the compound of Formula (IC) to be applied to an animal, premises or outdoor areas will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% w/w except for undiluted formulations such as pour-on formulations which in general will be deposited at a concentration in the range from 0.1 to 20.0% and preferably 0.1 to 10%. The amount of compound to be applied to stored products in general will lie in the range of from 0.1 to 20ppm. Space sprays may be applied to give an average initial concentration of 0.001 to 1mg of compound of formula (IC) per cubic metre of treated space.

The compounds of formula (IC) are also of use in the protection and treatment of plant species, in which case an effective insecticidal, acaricidal or nematocidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/Ha. Typical formulations for agricultural use contain between 0.0001% and 50% of a compound of formula (IC) and conveniently between 0.1 and 15% by weight of a compound of the formula (IC).

Dusts, greases, pastes and aerosol formulations are usually applied in a random fashion as described above and concentrations of 0.001 to 20% w/v of a compound of Formula (IC) in the applied formulation may be used.

The compounds of formula (IC) have been found to have activity against the common housefly (Musca domestica). In addition, certain compounds of formula (IC) have activity against other arthropod pests including Myzus persicae, Tetranychus urticae. Plutella xylostella, Culex spp. Tribolium castaneum, Sitophilus granarius, Periplaneta amiercana and Blattella germanica. The compounds of formula (IC) are thus useful in the control of arthropods e.g. insects and acarines in any environment where these constitute pests, e.g. in agriculture, in animal husbandry, in public health control and in domestic situations.

Insect pests include members of the orders Coleoptera (e.g. Anobium,Ceutorhynchus,Rhynchophorus. Cosmopolites, Lissorhoptrus. Meligethes, Hynothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus or Anthrenus spp.), Lepidoptera (e.g. Ephestia, Mamestra, Earias, Pectinophora, Ostrinia, Trichoplusia, Pieris, Laphygma, Aerotis, Amathes, Wiseana, Tryporysa, Diatraea, Sporganothis, Cydia, Archips, Plutella, Chilo, Heliothis, Spodoptera or Tineola spp.), Diptera (e.g. Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza and Melophagus spp.), Phthiraptera (Malonhaga e.g. Damalina spp. and Anoplura e.g. Linognathus and Haematopinus spp.), Hemiptera (e.g. Aphis, Bemisia,Phorodon, Aeneolamia, Empoasca, Parkinsiella, Pyrilla, Aonidiella, Coccus, Pseudococus, Helopeltis, Lygus, Dysdercus, Oxycarenus, Nezara, Aleurodes, Triatoma, Psylla, Mysus, Megoura, Phylloxera, Adelyes, Niloparyata, Nephrotetix or Cimex spp.), Orthoptera (e.g. Locusta, Gryllus, Schistocerca or Acheta spp.), Dictyoptera (e.g. Blattella, Periplaneta or Blatta spp.), Hymenoptera (e.g. Athalia, Cephus, Atta, Solenopsis or Monomorium spp.), Isoptera (e.g. Odontotermes and Reticulitermes spp.), Siphonaptera (e.g. Ctenocephalides or Pulex spp.), Thysanura (e.g. Lepisma spp.), Dermaptera (e.g. Forficula spp.), Pscoptera (e.g. Periosocus spp.) and Thysanoptera (e.g. Thrips tabaci),.

Acarine pests include ticks, e.g. members of the genera Boophilus,Ornithodorus, Rhipicephalus, Amblyomma, Hyalomma, Ixodes, Haemaphysalis, Dermacentor and Anocentor, and mites and manges such as Acarus, Tetranychus, Psoroptes, Notoednes, Sarcoptes, Psorergates, Chorioptes, Eutrombicula, Demodex, Panonychus, Bryobia, Eriophyes, Blaniulus, Polyphagotarsonemus, Scutigerella, and Oniscus spp.

Nematodes which attack plants and trees of importance to agriculture, forestry, horticulture either directly or by spreading bacterial, viral, mycoplasma or, fungal diseases of the plants, include root-knot nematodes such as Meloidogyne spp. (e.g. M. incognita); cyst nematodes such as Globodera spp. (e.g. G. rostochiensis); Heterodera spp. (e.g. H. avenae): Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g.R. reniformis); Rotylenchus spp. (e.g. R. robustus); Helicotylenchus spp. (e.g. H. multicinctus);Hemicycliophora spp. (e.g. H. gracilis); Criconemoides spp. (e.g. C. similis); Trichodorus spp. (e.g. T primitivus): dagger nematodes such as Xiphinema spp. (e.g. X. diversicaudatum), Longidorus spp (e.g. L. elongatus); Hoplolaimus spp. (e.g. H. coronatus); Aphelenchoides spp. (e.g. A. ritzema-bosi, A. besseyi): stem and bulb eelworms such as Ditylenchus spp. (e.g. D. dipsaci).

Compounds of the invention may be combined with one or more other pesticidally active ingredients (for example pyrethroids, carbamates and organophosphates) and/or with attractants, repellents, bacteriocides, fungicides, nematocides, anthelmintics and the like. Furthermore, it has been found that the activity of the compounds of the invention may be enhanced by the addition of a synergist or potentiator, for example: one of the oxidase inhibitor class of synergists, such as piperonyl butoxide or propyl 2-propynylphenylphosphonate; a second compound of the invention; or a pyrethroid pesticidal compound. When an oxidase inhibitor synergist is present in a formula of the invention, the ratio of synergist to compound of Formula (IC) will be in the range 25:1-1:25 eg about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilises and as scavengers.

The following Examples illustrate preferred aspects of the invention. All temperatures are in degrees Celsius.

## Experimental Procedures

### Procedure A

(i) 4-(2,2-Dibromovinyl)cyclohexanecarbonyl chloride (0.91 g) in dry dichloromethane (5 ml) was added to a cooled (0°C) solution of 3-propyl-3-hydroxymethyloxetane (0.406 g) and pyridine (0.64 ml) in dry dichloromethane (10 ml) under a stream of nitrogen. The reaction mixture was allowed to warm to room

temperature and to stir overnight. Further dichloromethane was added. The organic phase was then washed with dilute hydrochloric acid, saturated sodium bicarbonate solution and brine before drying over anhydrous magnesium sulphate. The solvent was evaporated in vacuo and the residue was purified by column chromatography on silica gel, pre-eluted with hexane containing 1% triethylamine. Elution with hexane/ether (3:1) gave 3-propyloxetan-3-ylmethyl 4-(2,2-dibromovinyl)cyclohexanecarboxylate (0.6 g) as a mixture of cis- and trans-isomers.

Infrared Spectrum (IR)(liquid film), 1722 (s) cm -1

Mass spectrum (MS), chemical ionisation, 2 peaks in glc/ms in a ratio of 3 : 1, both M+1 423.

(ii) Boron trifluoride etherate (40 µl) was added to a stirred solution of 3-propyloxetan-3-ylmethyl 4-(2,2-dibromovinyl)cyclohexanecarboxylate (0.57 g) in dry dichloromethane (10 ml) at -70°C. The mixture was allowed to warm to room temperature over 16 hours. Triethylamine (0.6ml) was then added. The organic phase was washed with brine before drying over anhydrous magnesium sulphate. The solvent was evaporated in vacuo and the residue was purified by column chromatography on alumina eluting with 3 : 7 dichloromethane:hexane saturated with ammonia. 1-[4-(2,2-Dibromovinyl)cyclohexyl]-4-propyl -2,6,7-trioxabicyclo[2.2.2]octane was obtained as a colourless oil (0.34 g).

Infrared spectrum (IR)(liquid film), 1060, 1020 $cm^{-1}$

Mass spectrum (MS), chemical ionisation, 2 peaks in glc/ms in a ratio of 3 : 1, both M+1 423.

Procedure B

(i) A solution of dimethyl sulphoxide (12ml) in dry dichloromethane (4.0ml) was added to a solution of oxalyl chloride (7.4ml) in dichloromethane (25ml) stirred at -70° under nitrogen. After the addition was complete the resulting mixture was stirred for a further 5 minutes at -70° before a solution of 3-hydroxymethyl-3-n-propyl oxetane (10.0g) in dichloromethane (25ml) was added, dropwise, over 10 minutes. The resulting mixture was allowed to stir for a further 30 minutes when neat triethylamine (54ml) was added over approximately 30 minutes. The reaction mixture was allowed to warm to room temperature over 3 hours when it was poured into water. The organic phase was separated and the aqueous layer was further extracted with dichloromethane. The combined organic extracts were washed with dilute hydrochloric acid, saturated sodium bicarbonate and brine. The resulting organic phase was dried over anhydrous magnesium sulphate and evaporated in vacuo to give 3-formyl-3-n-propyloxetane (10.5g) (European Patent Application No. 216624) as a yellow oil.

(ii) A mixture of hex-5-ynoic acid (1g) and thionyl chloride (1.95ml) in benzene (25 ml) was heated at reflux for 3 hours. The resulting solution was allowed to cool and then evaporated in vacuo. The acid chloride thus obtained was added to a stirred solution of 3-formyl-3-n-propyloxetane (1.14g) in ether (50ml) followed by a solution of sodium cyanide (0.61g) in water (1ml). The resulting mixture was stirred briskly at room temperature for 16 hours. After this time the reaction mixture was washed with water, saturated sodium bicarbonate and brine, before drying over anhydrous magnesium sulphate. The solvent was removed in vacuo and the residue was purified by column chromatography on silica, pre-eluted with hexane containing 1% triethylamine. Gradient elution with hexane/ether mixtures gave 1-cyano-1-(3-propyloxetan-3-yl) methyl hex-5-ynoate as a colourless oil (1.2g).

Gas-liquid chromatography (g.l.c): OV-17 at 175° produced one peak.

Nuclear magnetic resonance spectrum (NMR) was as follows:$^1$H (p.p.m from TMS in $CDCl_3$, integral, number of peaks): 5.6, 1H, s; 4.75-4.4, 4H, m; 2.8-0.9, 14H, m.

(iii)Boron trifluoride etherate (0.25ml) was added to a solution of 1-cyano-1-(3-propyloxetan-3-yl)methyl hex-5-ynoate (0.5 g) in dry dichloromethane (10ml) stirred at -70° under a nitrogen atmosphere. The resulting solution was allowed to warm to room temperature overnight. Triethylamine (0.38ml.) was added and the solvent was removed under vacuum. The residue was partitioned between water and diethyl ether. The organic phase was separated and washed with water and brine before drying over anhydrous magnesium sulphate and evaporation in vacuo. The residue was purified by column chromatography on alumina eluting with 1:4 dichloromethane:hexane saturated with ammonia. 1-(Pent-4-ynyl)-4-propyl-2,6,7-trioxabicyclo [2.2.2]octane-3-carbonitrile was obtained as an oil (0.25g).

Gas-liquid chromatography (g.l.c): OV-17 at 175° produced one peak.

Procedure C

(i) A solution of ethyl (E)-hept-2-en-6-ynoate (1.8g) in 50% aqueous methanol containing 5% sodium hydroxide was stirred at ambient temperature overnight. The methanol was removed under reduced pressure and the resulting aqueous phase was extracted with dichloromethane. The aqueous phase was

adjusted to pH 1 with concentrated hydrochloric acid and re-extracted with dichloromethane. These organic extracts were washed with brine, dried over anhydous magnesium sulphate and then evaporated in vacuo to leave (E)-hept-2-en-6-ynoic acid as a white solid (1.3g).

Procedure D

(i) A solution of 3-mercaptopropionic acid (15 ml) in dry dimethylformamide (250 ml) was stirred at 0°, under nitrogen. Sodium hydride (10.2 g; 80% dispersion in oil) was added carefully and the mixture was stirred at 40° for 1 hour. The mixture was cooled to 0°C and propargyl bromide (60g; 80% in toluene) was added dropwise. The mixture was stirred at 20° for 3 hours and then at 80° for 1 hour. The mixture was cooled and poured into water. The aqueous mixture was extracted with diethyl ether, and the extracts washed with water and dried over anhydrous magnesium sulphate. The solvent was removed in vacuo. The resulting oil (30g) (mainly prop-2-ynyl 3-(prop-2-ynyl-thio)propionate) was added to a solution of sodium hydroxide (8.0g) in water (100ml) and methanol (100ml) and the mixture was stirred at 20° for 24 hours. The mixture was extracted with diethyl ether and the aqueous solution was acidified with hydrochloric acid. The acidic mixture was extracted with diethyl ether and the ethereal extracts were dried over anhydrous magnesium sulphate. The solvent was removed in vacuo. 3-(Prop-2-ynylthio) propionic acid was obtained as a red oil (12.0g) and was used without further purification.

(ii) 4-n-Propyl-1-[2-(prop-2-ynylthio)ethyl]-2,6,7-trioxabicyclo[2.2.2]octane was prepared from 3-(prop-2-ynylthio)propionic acid and 3-hydroxymethyl-3-n-propyloxetane using methodology described in Example I.

Procedure E

(i) A mixture of hex-5-ynoic acid (1g) and thionyl chloride (1.95ml) in benzene (25ml) was heated under reflux for 3 hours. The resulting solution was cooled and then evaporated in vacuo. The acid halide thus obtained was taken up in ether (5ml) and added, dropwise, to a stirred solution of 3-hydroxymethyl-3-n-propyloxetane (1.2g) and pyridine (0.8ml) in dry ether (20ml.). The reaction mixture was stirred at room temperature for 16 hours. After this time the organic phase was washed with water, 5% hydrochloric acid, saturated sodium bicarbonate solution and brine before drying over anhydrous magnesium sulphate and then evaporation in vacuo. The residue was purified by chromatography on silica, pre-eluted with hexane containing 1% triethylamine. Elution with hexane/ether mixtures gave (3-propyloxetan-3-yl)methyl hex-5-ynoate (1.33g) as a colourless oil.

Gas-liquid chromatography (g.l.c.): OV-17 at 175° produced one peak.

Nuclear magnetic resonance spectrum (NMR) was as follows: $^1H$ (p.p.m. from TMS in $CDCl_3$, integral, number of peaks,): 4.45, 4H, s; 4.20, 2H, s; 2.8-0.8, 14H, m.

(ii) Boron trifluoride etherate (0.18ml) was added to a stirred solution of (3-propyloxetan-3-yl)methyl hex-5-ynoate (1.33g.) in dry dichloromethane (25ml) at -70°. The mixture was allowed to warm to room temperature over 16 hours. Triethylamine (0.28ml) was then added and the solvent was removed in vacuo. The residue was partitioned between diethyl ether and water. The organic phase was separated and further washed with water and brine before drying over anhydrous magnesium sulphate. The solvent was evaporated in vacuo and the residue was purified by column chromatography on alumina eluting with 1 : 6 dichloromethane : hexane saturated with ammonia. 1-(Pent-4-ynyl)-4-propyl-2,6,7-trioxabicyclo-[2.2.2]octane was obtained as a colourless oil which crystallised on trituration (0.64g) with hexane.

Gas-liquid chromatography (g.l.c): OV-17 at 200° produced one peak.

Example I

4-t-Butyl-1-(3,3-dimethylbut-1-ynyl)-2,6,7-trioxabicyclo[2.2.2]octane

(i) Using the method described in stage (i) of Procedure A and starting from 4,4-dimethyl-2-pentynoyl chloride (A R Katritsky et al, J Chem. Soc., Perkin Trans 2, 1974, 282), and 3-t-butyl-3-hydroxymethyloxetane, (3-t-butyloxetan-3-yl)methyl 4,4-dimethylpent-2-ynoate was obtained as a solid.

Gas-liquid chromatography (glc) : OV17 at 180°C produced one peak.

(ii) Using the method described in Stage (ii) of Procedure A and starting from (3-t-butyloxetan-3-yl)methyl 4,4-dimethylpent-2-ynoate, 4-t-butyl-1-(3,3-dimethylbut-1-ynyl)-2,6,7-trioxabicyclo[2.2.2]octane was obtained as colourless crystals (m pt 204 - 206 °C)

Gas-liquid chromatography (glc): OV17 at 180° produced one peak.

Using the methodology described in Procedure B and starting from 3-formyl-3-n-propyloxetane and 3-i-butyl-3-formyloxetane, 1-(3,3-dimethylbut-1-ynyl)-4-n-propyl-2,6,7-trioxabicyclo[2.2.2]octane-3-carbonitrile and 4-i-butyl-1-(3,3-dimethylbut-1-ynyl)-2,6,7-trioxabicyclo[2.2.2]octane-3-carbonitrile were prepared.

Using analogous methodology E-1-(3,3-dimethylbut-1-enyl)-4-n-propyl-2,6,7-trioxabicyclo[2.2.2]-octane-3-carbonitrile and trans-1-(2-t-butylcyclopropyl)-4-n-propyl-2,6,7-trioxabicyclo[2.2.2]octane-3-carbonitrile were prepared from E-4,4-dimethylpent-2-enoic acid and ethyl trans-2-t-butylcyclopropane carboxylate (E.L.Foreman and S.M.McElvain J.Amer. Chem. Soc., 1940, 62, 1438 and I.A. D'yakonov et al Chem.Abs. 70:78062m respectively).

Using analogous methodology 4-t-butyl-1-(3,3-dimethylbutyl)-2,6,7-trioxabicyclo[2.2.2]octane and 1-(3,3-dimethylbutyl)-4-n-propyl-2,6,7-trioxabicyclo[2.2.2]octane-3-carbonitrile were prepared from 4,4-dimethylpentanoic acid (G.M.Whitesides et al J. Amer. Chem. Soc. 1967, 89, 1135).

## Example II

### 1-[(Z)-1-Fluoro-3,3-dimethylbut-1-enyl]-4-propyl-2,6,7-trioxabicyclo [2.2.2] octane-3-carbonitrile

i) A mixture of ethyl bromofluoroacetate (25g) (Fluorochem.) and triethylphosphite (30ml) was heated at 150°, in an apparatus set-up for fractional distillation, until production of bromoethane ceased. Distillation of the residue, under reduced pressure, then gave ethyl diethylphosphonofluoroacetate as a colourless oil (9.05g, b.p. 80-88°, 0.1mm Hg).

ii) n-Butyllithium (4.8ml of a 1.6M solution in hexane) was added to a stirred solution of diisopropylamine (1.1ml) in tetrahydrofuran (15ml) at 0°C under nitrogen. The resulting mixture was maintained at 0°C for 0.5 hour and then cooled to -70° when a solution of ethyl diethylphosphonofluoroacetate (1.7g) in tetrahydrofuran (5ml) was added. After a further 0.5 hour at -70°,trimethylacetaldehyde (0.76ml) was added neat and the resulting mixture was allowed to warm to room temperature over 5 hours. After this time, water (5ml) was added and the bulk of the solvent was removed under reduced pressure. The residue was partitioned between diethyl ether and water. The organic phase was separated, washed with water, 10% hydrochloric acid solution and brine before drying over anhydrous magnesium sulphate. The solvent was removed in vacuo to leave ethyl (Z)-2-Fluoro-4,4-dimethylpent-2-enoate as a pale green oil (1.0g).

Gas-liquid chromatography (glc): OV-17 at 100° produced one peak.

iii) Using the method described in Procedure C, stage (i) (Z)-2-Fluoro-4,4-dimethylpent-2-enoic acid was prepared from ethyl (Z)-2-fluoro-4,4-dimethylpent-2-enoate.

iv) Using the method described in Procedure B stages ii) and iii), 1-[(Z)-1-Fluoro-3,3-dimethylbut-1-enyl]-4-propyl-2,6,7-trioxabicyclo[2.2.2]octane-3-carbonitrile was prepared from 3-formyl-3-n-propyl-oxetane and (Z)-2-fluoro-4,4-dimethylpent-2-enoic acid.

Gas liquid chromatography (glc): OV-17 at 250° produced one peak.

## Example III

### 1-(t-Butylthiomethyl)-4-n-propyl-2,6,7-trioxabicyclo[2.2.2]octane-3-carbonitrile

Using stages (i) and (ii) of Procedure D and starting from t-butylthiol and ethyl bromoacetate t-butylthioacetic acid was prepared.

1-(t-butylthiomethyl)-4-n-propyl-2,6,7-trioxabicyclo[2.2.2]octane-3-carbonitrile was prepared from t-butylthioacetic acid and 3-formyl-3-n-propyl- oxetane using methodology described in Procedure B.

Formulations

1. Emulsifiable Concentrate

| | |
|---|---|
| Compound of formula (I) | 10.00 |
| Ethylan KEO | 20.00 |
| Xylene | 67.50 |
| Butylated Hydroxyanisole | 2.50 |
| | 100.00 |

2. Wettable Powder

| | |
|---|---|
| Compound of formula (I) | 25.00 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

3. Dust

| | |
|---|---|
| Compound of formula (I) | 0.50 |
| Butylated Hydroxyanisole | 0.10 |
| Talc | 99.40 |
| | 100.00 |

4. Bait

| | |
|---|---|
| Compound of formula (I) | 40.25 |
| Icing Sugar | 59.65 |
| Butylated hydroxy toluene | 0.10 |
| | 100.00 |

5. Lacquer

| | |
|---|---|
| Compound of formula (I) | 0.1 |
| Piperonyl Butoxide | 0.5 |
| Butylated Hydroxyanisole | 10.1 |
| High aromatic white spirit | 92.0 |
| | 100.00 |

6. Aerosol

| | |
|---|---|
| Compound of formula (I) | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

7. Spray

| | |
|---|---|
| Compound of formula (I) | 0.1 |
| Butylated Hydroxyanisole | 0.1 |
| Xylene | 10.0 |
| Odourless Kerosene | 89.8 |
| | 100.00 |

8. Potentiated Spray

| | |
|---|---|
| Compound of formula (I) | 0.1 |
| Piperonyl Butoxide | 0.5 |
| Butylated Hydroxyanisole | 0.1 |
| Xylene | 10.1 |
| Odourless Kerosene | 89.2 |
| | 100.00 |

## BIOLOGICAL ACTIVITIES

The following examples illustrate, in a non-limiting manner, the pesticidal activity of compounds of formula (I).

### Spray Tests

The activity of the compounds of the invention were tested by dissolving the compounds in acetone (5%) and then diluting in water: 'Synperonic' (94.5%: 0.5%) to give a water emulsion. The solution was then used to treat the following insects.

### Musca domestica

20 female Musca were contained in a cardboard cylinder with gauze over both ends. Solution containing the compound was sprayed onto the insects so enclosed and mortality assessed after 48 hours at 25°.

The following compound was active at <1000p.p.m.

4.

The following compounds were active at <200p.p.m.

1, 2, 3.

Sitophilus granarius and Tribolium castaneum

20 adult Sitophilus and Tribolium were added to 10g wheat which had been previously treated with 2ml of the solution containing the compounds. Mortality was assessed after 6 days at 25°C.

The following compounds were active against Sitophilus granarius at <1000 p.p.m.:
3, 4.

The following compound was active against Tribolium castaneum at <1000 p.p.m.:-
2.

Myzus persicae

10 adult Myzus were placed on a leaf disc of chinese cabbage. 24 hours later the disc was sprayed with a solution containing the compound. Mortality was assessed after 2 days at 25°.

The following compound was active at <1000 p.p.m.:-
2.

Plutella xylostella

7 Plutella larvae were sprayed with the solution containing the compound and added to a chinese cabbage leaf which had been similarly sprayed and left to dry. Alternatively 8-10 Plutella larvae were put onto leaf discs and sprayed with the solution containing the compound. Mortality was assessed after 2 days at 25°.

The following compounds were active at <1000 p.p.m.:-
1, 3, 5.

Tetranychus urticae

Leaf discs containing mixed population of Tetranychus urticae were sprayed with the solution of the compound. Mortality was assessed after 2 days at 25°C.

The following compound was active at <1000 p.p.m.:-
1.

Topical Application Tests

The activity of compounds of the invention against unanaesthatised female Musca domestica (WRL strain) was demonstrated by the topical application to the test insect of a solution of the compound under test with piperonyl butoxide in butanone. Mortality was assessed at 48 hours.

The following compounds were active at 1µg:-
1, 2, 3.

The activity of compounds of the invention against anaesthetised male Periplaneta americana was demonstrated by the topical application to the test insect of a solution of the compound under test in butanone. Mortality was assessed after 6 days.

The following compound was active at <50µg:-
1.

The activity of compounds of the invention against anaesthetised male Blattella germanica was demonstrated by topical application to the test insect of a solution of the compound under test in butanone. Mortality was assessed after 6 days.

The following compounds were active at <5µg:-
1, 2.

APPENDIX 1

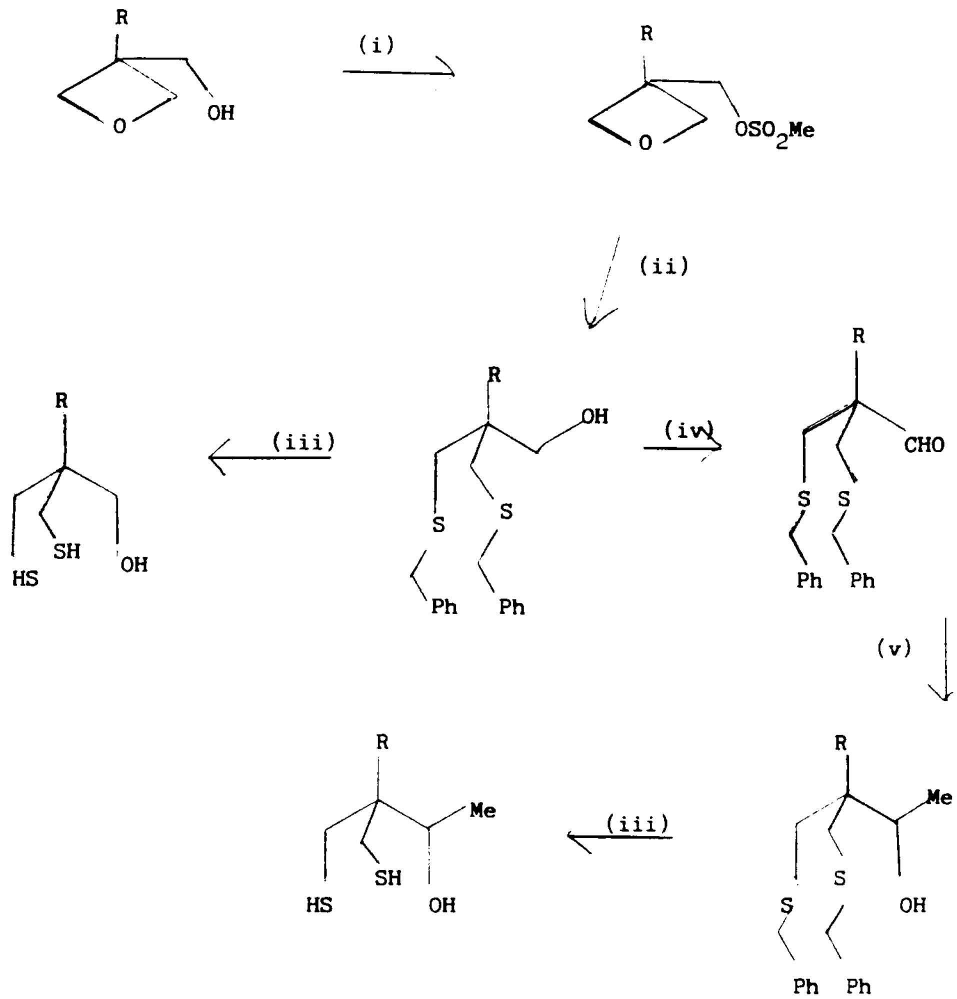


(i) $MeSO_2Cl$, pyridine (ii) $PhCH_2SH$, NaH, Dimethylformamide

(iii) Na, liquid $NH_3$ (iv) Pyridinium chlorochromate, sodium acetate, $CH_2Cl_2$

(v) MeMgI, $Et_2O$

Table I - Trioxabicyclo-octanes

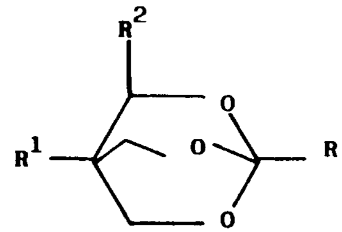

| Compound No. | R | $R^{1b}$ | $R^{2b}$ | Synthetic Method Example |
|---|---|---|---|---|
| 1 | 3,3-dimethylbutyl | t-Bu | H | I |
| 2 | 3,3-dimethylbutyl | n-pr | CN | I |
| 3 | E-3,3-dimethylbut-1-enyl | n-pr | CN | I |
| 4 | Z-3,3-dimethyl-1-fluoro-but-1-enyl | n-pr | CN | II |
| 5 | t-butylthiomethyl | n-pr | CN | III |

Table II - Characterising Data for Trioxabicyclo-octanes

| Compound No. | mpt | Mass Spectrum Chemical Ionisation M + 1 | Nuclear Magnetic Resonance Spectrum $^1H$ (ppm from TMS in $CDCl_3$, integral, multiplicity, $J_{mz}$) |
|---|---|---|---|
| 1 | 169° | 257 | 4.00,6H,S; 1.62,2H,m; 1.32,2H,m; 0.87,9H,S; 0.85,9H,S. |
| 2 | 54° | 268 | 4.77,1H,d; 4.20,1H,m; 3.95,3H,m; 1.70,2H,m; 1.35,6H,m; 0.95,3M,t; 0.85,9H,S. |
| 3 | 77.5 | 266 | 6.15,1H,d; 5.35,1H,d; 4.82,1H,d; 4.23,1H,m; 4.05,3H,m; 1.30,4H,m; 1.00,9H,S; 0.95,3H,t. |
| 4 | Oil | 284 | 5.50,1H,d,$J_{hz}$ 9 and 3; 4.06,3H,m; 1.5-1.2, 4H,m; 1.15,9H,S; 0.95,3H,t,$J_{hz}$7. |
| 5 | Oil | 286 | 4.8,1H,m; 4.3-4.0,4H,m; 2.8,2H,S; 1.5-1.2, 13H,m; 0.95,3H,m. |

## Claims

1. A compound of the formula (IC):

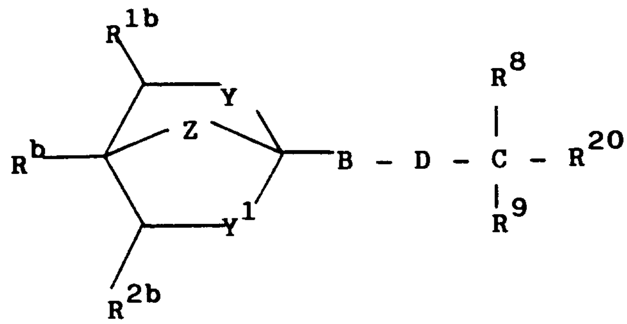

(Ic)

wherein $R^b$ is a $C_{2-10}$ non-aromatic hydrocarbyl group optionally substituted by, cyano, halo, $C_{1-4}$ alkoxy, or a group $S(O)_mbR^{3b}$ where $R^{3b}$ is $C_{1-4}$ alkyl and $m^b$ is 0, 1 or 2, or $R^b$ is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mbR^{3b}$ as defined hereinbefore;
$R^{1b}$ and $R^{2b}$ may be the same or different, and each is hydrogen, halo, or a $C_{1-3}$ aliphatic group optionally substituted by halo, cyano, $C_{1-5}$ carbalkoxy, $C_{1-4}$ alkoxy, or a group $S(O)_{m'}R^4$ wherein m' is 0, 1 or 2 and $R^4$ is $C_{1-4}$ alkyl; $C_{2-3}$ alkynyl, cyano, gem dimethyl, or $C_{1-5}$ carbalkoxy, or $R^{1b}$ and $R^b$ and the carbon atoms to which they are attached form a $C_{5-7}$ carbocyclic ring optionally substituted by halo, or a $C_{1-3}$ alkyl or alkoxy or $C_{2-3}$ alkenyl;
B is a single bond, methylene or a $C_{2-6}$ aliphatic chain which may contain one or two heteroatoms selected from oxygen and sulphur and/or double bonds but not triple bonds interspersed in the chain and which may be substituted by one to four substituents which may be the same or different and are each independently selected from hydroxy, oxo, halo, $C_{1-4}$ alkyl, $C_{1-4}$ alkoxy, $C_{1-4}$acyloxy, epoxy, a $C_{1-4}$ alkylidene group, a $C_{1-6}$ carbalkoxy group, $C_{1-4}$ haloalkyl or cyano;
D is a single bond or a group $CH_2O$, $CH_2S(O)_n$ wherein n is 0, 1 or 2 or D is a 1, 2 cyclopropyl group;
$R^8$, $R^9$ and $R^{20}$ may be the same or different and are selected from chloro, bromo, methoxy or methyl optionally substituted by methoxy or fluoro;
Y and $Y^1$ are the same or different and are each selected from oxygen and $S(O)_{n'}$ where n' is 0, 1 or 2; and Z is $CH_2CH_2$, $CH_2O$ or $CH_2S(O)_{n''}$, wherein n'' is 0, 1 or 2, provided that B cannot be a single bond or methylene group when D is a single bond.

2. A compound of the formula (IC) according to claim 1 wherein $R^b$ is n-propyl, n-butyl i-butyl or t-butyl.

3. A compound of the formula (IC) according to claim 1 or 2 wherein $R^{1b}$ and $R^{2b}$ are independently selected from hydrogen, methyl, cyano or trifluoromethyl.

4. A compound selected from:
4-t-Butyl-1-(3,3-dimethylbutyl)-2,6,7-trioxabicyclo[2.2.2]octane
1-(3,3-Dimethylbutyl)-4-propyl-2,6,7-trioxabicyclo[2.2.2]octane-3-carbonitrile
1-[(E)-3,3-Dimethylbut-1-enyl]-4-propyl-2,6,7-trioxabicyclo[2.2.2]octane-3-carbonitrile
1-(t-Butylthiomethyl)-4-propyl-2,6,7-trioxabicyclo[2.2.2]octane-3-carbonitrile
1-[z)-1-Fluoro-3,3-dimethylbut-1-enyl]-4-propyl-2,6,7-trioxabicyclo[2.2.2]octane-3-carbonitrile

5. A pesticidal formulation comprising a compound of the formula (IC) as defined according to any of the preceding claims in admixture with one or more carriers or diluents.

6. A pesticidal formulation according to claim 5 which additionally contains a synergist or potentiator.

7. A pesticidal formulation according to either claim 5 or claim 6 which additionally contains one or more pesticidally active ingredients, attractants, repellents, bacteriocides, fungicides and/or anthelmintics.

8. A compound of the formula (IC) for use in human or veterinary medicine.

9. A method for the control of arthropod or helminth pests which comprises administering to the arthropod or helminth or their environment an effective amount of a compound of the formula (IC) as prepared

according to any one of the preceding claims.

**10.** A method for the control of pesticidal infestations on plants and/or stored products and/or an environment which comprises administering an effective amount of a compound of the formula (IC) as defined according to any one of the preceding claims to the plant and/or stored product and/or an environment susceptible to pest infestation.

**11.** A process for the manufacture of a compound of the formula (IC) as defined according to claim 1 which comprises:

i) for the production of compounds wherein Y and $Y^1$ are oxygen and Z is $CH_2O$:

a) the cyclisation of a compound of the formula (II):

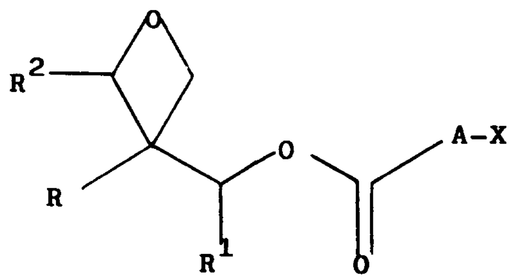

(II)

wherein $R^b$ to $R^{2b}$, $R^8$, $R^9$, $R^{20}$, B and D are as defined according to claim 1, in the presence of an acid catalyst;

ii) for the production of compounds wherein n' is O, $Y^1$ is O or S, Y is O or S, Z is $CH_2S$ or $CH_2O$, the reaction of a compound

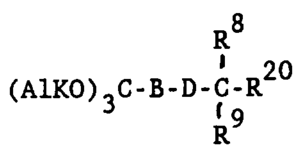

with a compound of the formula XIV):

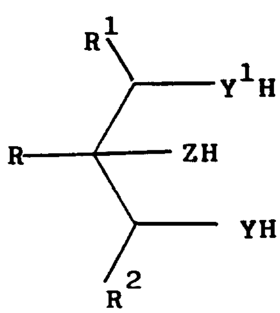

(XIV)

wherein $R^b$ to $R^{2b}$, $R^8$, $R^9$, $R^{20}$, B, D, Y, $Y^1$ and Z are as defined according to claim 1 and Alk is a $C_{1-4}$ alkyl group or;

iii) for the production of compounds wherein $Z = CH_2S$ or $CH_2O$ and Y and $Y^1$ are sulphur the reaction of a compound

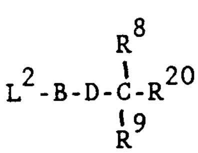

with a compound of the formula (XV):

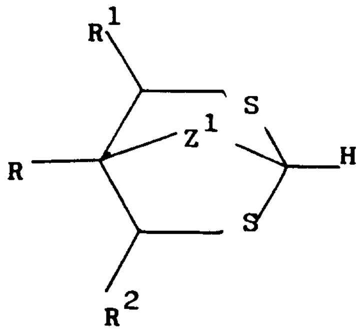

(XV)

wherein Rb to $R^{2b}$, $R^8$, $R^9$, $R^{20}$, B and D are as defined according to claim 1, $Z^1$ is $CH_2S$ or $CH_2O$ and $L^2$ is a leaving group, or;
iv) for the production of compounds wherein Y, $Y^1$ are O and Z is $CH_2CH_2$, the reaction of a compound of the formula (XI) with acid:

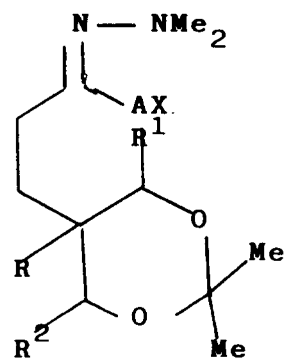

(XVI)

wherein $R^b$ to $R^{2b}$, $R^8$, $R^9$, $R^{20}$, B and D are as hereinbefore defined or;
v) the conversion of a compound of the formula (IC) into another compound of the formula (IC).

European Patent Office

# EUROPEAN SEARCH REPORT

Application Number

EP 93 10 0641

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 216 625 (WELLCOME)<br>* claims 1,12,16 *<br>----- | 1,5,8 | C07D493/08<br>C07D497/08<br>C07D495/08<br>A01N43/90<br>A61K31/335<br>A61K31/385<br>A61K31/39<br>//(C07D493/08, 319:00,319:00)<br>(C07D497/08, 327:00,327:00)<br>(C07D497/08, 339:00,327:00)<br>(C07495/08, 339:00,339:00)<br>(C07D493/08, 319:00,311:00)<br>(C07497/08, 335:00,327:00) |
| | | | TECHNICAL FIELDS SEARCHED (Int. Cl.4) |
| | | | C07D<br>A01N<br>A61K |

The present search report has been drawn up for all claims

| Place of search | Date of completion of the search | Examiner |
|---|---|---|
| THE HAGUE | 02 MARCH 1993 | ALFARO FAUS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document